# EUROPEAN PATENT APPLICATION

(11) **EP 4 578 422 A1**
(43) Date of publication of application: **02.07.2025**
(21) Application number: 24220919.5
(22) Date of filing: 18.12.2024
(51) Int. Cl.: A61B 90/70, F16J 15/00, F16J 15/3232, A61L 2/07

(54) **SEALING APPARATUS AND CORRESPONDING OPERATING METHOD**

(30) Priority: 27.12.2023 IT 202300028107
(71) Applicant: Icos Pharma S.p.A., 33080 Zoppola (IT)
(72) Inventor: Beni, Stefano, 33170 Pordenone (IT); Bertolini, Gianni, 33078 San Vito al Tagliamento (PN) (IT)
(74) Representative: Petraz, Gilberto Luigi

(57) **Abstract**

Hermetic sealing apparatus (10) comprising both a support frame (11) provided with a first aperture (13) delimited by first perimeter edges (15) and able to be perimetrically and hermetically attached to a dividing wall (100) which separates a reception zone (101) for receiving contaminated instruments to be treated from a sterile zone (102), and also a central rim (12) perimetrically and hermetically associated with the first perimeter edges (15) and provided with second perimeter edges (17) facing the latter, and with a second aperture (16) hermetically connectable to a treatment chamber (105) of said sterile zone (102). At least two sealing gaskets (19, 20) are attached between the first (15) and second (17) edges, disposed in series and engaged by attachment elements (21) so as to define a hermetically closed isolation chamber (35).

## Description

### FIELD OF THE INVENTION

The present invention concerns a sealing apparatus, preferably with double gasket, and the corresponding operating method. The hermetic sealing apparatus is associated with a dividing wall and has an interface function between a reception zone for receiving instruments to be treated, for example surgical or laboratory instruments, and a sterile zone which comprises, for example, a treatment chamber of a sterilization machine.

### BACKGROUND OF THE INVENTION

For different laboratory and pharmaceutical applications, it is necessary to isolate and contain possible hazardous contaminating agents in an enclosed environment, in order to guarantee an adequate biosafety level (BSL - BioSafety Level). The biosafety level ranges from BSL-1, that is, the least hazardous level 1, to BSL-4, that is, the most hazardous level 4.

For this purpose, hermetic sealing apparatuses attached to a dividing wall and having an interface function between a reception zone for receiving instruments to be treated, which may already have been pre-treated but are still contaminated, and a sterile zone are known. In the sterile zone there is usually a treatment chamber of a machine for sterilizing the instruments, such as an autoclave, which is hermetically associated with the sealing apparatus.

The sealing apparatus can comprise a structure defined by a support frame provided with a first aperture and perimetrically and hermetically associated with the dividing wall, and by a central rim perimetrically and hermetically associated with the first aperture and provided with a second aperture hermetically connected to the treatment chamber.

Two flexible bio-containment gaskets are attached between the support frame and the central rim, in correspondence with respective reciprocally facing perimeter edges.

The two gaskets are disposed in series, and a closed isolation chamber is created between them to prevent the transfer of contaminating agents. Usually, one of the two gaskets is disposed on the side of the reception zone, and the other on the side of the sterile zone.

Bolts are used to attach the gaskets to the perimeter edges, which close the gaskets pack-wise and have a head end facing the reception zone and a terminal end where the clamping nut is located, the terminal end facing the sterile zone.

In order to guarantee the hermetic seal, these sealing assemblies also comprise generating means associated with the gaskets and able to generate an overpressure, that is, a positive pressure, inside the isolation chamber. This positive pressure is generated to prevent the possible passage of contaminating agents from the reception zone to the sterile zone through the isolation chamber itself.

Known sealing assemblies are described for example in US 2017/007730 A1, EP 3 919 086 A2 and WO 2022/131989 A1.

However, one disadvantage of these sealing assemblies is that the bolts pass from one side of the gaskets to another, and in the event that there is not a perfect seal between the isolation chamber and the bolts, the overpressure present inside the chamber may lead to the passage of contaminating agents from the reception zone to the sterile zone, for example in correspondence with the terminal end of the bolt present in the sterile zone.

This may result in other disadvantages related to the dispersion of such contaminating agents into zones that are not hermetically sealed.

There is therefore the need to perfect a hermetic sealing apparatus that can overcome at least one of the disadvantages of the state of the art.

To do this, it is necessary to solve the technical problem of providing a hermetic sealing apparatus that prevents the possible passage of contaminating agents toward the sterile zone, even if there is not a perfect seal between the bolts and the corresponding gasket.

One purpose of the present invention is to provide a hermetic sealing apparatus that is safe and simple to produce.

Another purpose of the present invention is to provide a hermetic sealing apparatus that is effective and that guarantees the hermetic seal while respecting a high level of biosafety.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes and to resolve the technical problem described above in a new and original way, also achieving considerable advantages compared to the state of the prior art, a sealing apparatus according to the present invention comprises both a support frame provided with a first aperture delimited by first perimeter edges, and also a central rim perimetrically and hermetically associated with the first perimeter edges.

The support frame is able to be perimetrically and hermetically attached in correspondence with an aperture of a dividing wall, which separates a reception zone for receiving contaminated instruments to be treated from a sterile zone which comprises a treatment chamber for the instruments. The instruments can be surgical or laboratory instruments.

The central rim is provided with second perimeter edges facing the first edges of the support frame, and with a second aperture hermetically connectable to the treatment chamber.

At least two sealing gaskets are attached between the first and second edges, disposed in series and engaged pack-wise by attachment elements, so as to define a hermetically closed isolation chamber between them.

In accordance with the present invention, the sealing apparatus comprises a device for generating a vacuum through Venturi, and a fluidic circulation circuit having a first duct hermetically associated with the chamber and able to fluidically connect the latter to the vacuum generating device, in order to generate a negative pressure therein able to draw any infiltrations of contaminating agents, expelling them into the reception zone. The sealing apparatus comprises: a first valve disposed along the first duct and able to open and close for the selective fluidic connection between the chamber and the vacuum generating device; a second valve disposed along a second duct connected to the vacuum generating device and able to open and close for the selective passage of a driving fluid toward the latter; and a pressure management device fluidically associated with the first duct between the chamber and the first valve, and operationally connected to the first and second valve, wherein the pressure management device is configured to open and close the first and second valve simultaneously, for the selective activation of the vacuum generating device.

Doing so achieves the advantage that, even if there is not a perfect seal between the gaskets and the head or terminal ends of the clamping elements, any contaminating agents are not dispersed in the sterile zone, but remain in the reception zone. Furthermore, the simultaneous drive of the valves increases the speed and efficiency of the vacuum generating device.

The vacuum generating device and the fluidic circulation circuit can be disposed in the reception zone.

In particular, the pressure management device is configured to be activated as a function of an activation threshold value correlated to a pressure present in the chamber.

The pressure management device can be a differential pressure switch able to be activated as a function of an activation threshold value given by a differential between a pressure in the chamber and a pressure in the reception zone.

The pressure management device can be capable of generating a maintenance intervention alarm if the vacuum generating device is not capable of restoring, after a certain interval of time, a suitable pressure in the chamber. This suitable pressure has to be such as to allow the pressure management device to be deactivated.

The second duct can comprise a narrowing nozzle which is inserted inside a connection chamber to which the first and second duct are hermetically connected and which, thanks to the section narrowing, allows to increase the speed of the flow of driving fluid toward a mixing zone in order to promote the Venturi effect.

The vacuum generating device can comprises a divergent part from which a mixing fluid flows out into the reception zone.

A silencing member can be positioned in correspondence with a terminal portion of the divergent part.

In accordance with one possible embodiment, the first and second edges can have profiles comprising an undercut protruding toward the reception zone and having respective abutment portions for corresponding external and internal perimeter zones of the gaskets which are clamped from one side to the other by the attachment elements, so that one of the two gaskets is disposed and facing toward the sterile zone and that corresponding head and terminal ends of the attachment elements are present in the reception zone and in the sterile zone, respectively.

In accordance with another possible embodiment, the first and second edges can have profiles each comprising at least two consecutive undercuts, protruding toward the reception zone and defining respective abutment portions for corresponding external and internal perimeter zones of the gaskets which are clamped from one side to the other by the attachment elements so that corresponding head and terminal ends of the latter remain in the reception zone.

In particular, the at least two undercuts of the first and second edges can be spaced apart from each other, defining a hollow space in which the gaskets are disposed, and are created in such a way as to define stepped profiles converging from the reception zone toward the sterile zone, so that the hollow space has a greater width toward the reception zone and a smaller width toward the sterile zone. Advantageously, the sealing apparatus allows to guarantee a biosafety level 4 (BSL-4).

In the reception zone, on opposite sides with respect to the hollow space, the undercuts can define respective collection compartments which allow to contain any leakages of contaminating agents from the terminal ends of the attachment elements.

The present invention also concerns a dividing wall comprising a sealing apparatus as disclosed above having an interface function between a reception zone for receiving contaminated instruments to be treated and a sterile zone comprising a treatment chamber of a machine for sterilizing the instruments.

The present invention also concerns a method of operation of a hermetic sealing apparatus as disclosed above. The method comprises at least a step of generating a vacuum in the chamber, in which the vacuum generating device generates a negative pressure in the chamber through Venturi effect, in order to draw any infiltrations of contaminating agents present therein and expel them into the reception zone.

In the vacuum generation step, a pressure management device is activated, in particular as a function of an activation threshold value correlated to a pressure in the chamber, and it opens a first and a second valve, thus activating the vacuum generating device, wherein the first valve is disposed along the first duct and opens and closes for the selective fluidic connection between the chamber and the vacuum generating device, and wherein the second valve is disposed along a second duct connected to the vacuum generating device and opens and closes for the selective passage of a driving fluid toward the latter. The driving fluid can be compressed air.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a partly sectioned top view of a hermetic sealing apparatus according to the present invention, which is hermetically associated with a dividing wall between a reception zone for receiving contaminated instruments to be treated, and a sterile zone in which there is a treatment chamber;
- fig. 2 is a partly sectioned front view of the sealing apparatus of fig. 1;
- fig. 3 is a detailed, partly sectioned top view of the sealing apparatus of fig. 1;
- fig. 4 is a detailed, partly sectioned top view of the sealing apparatus according to another embodiment.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

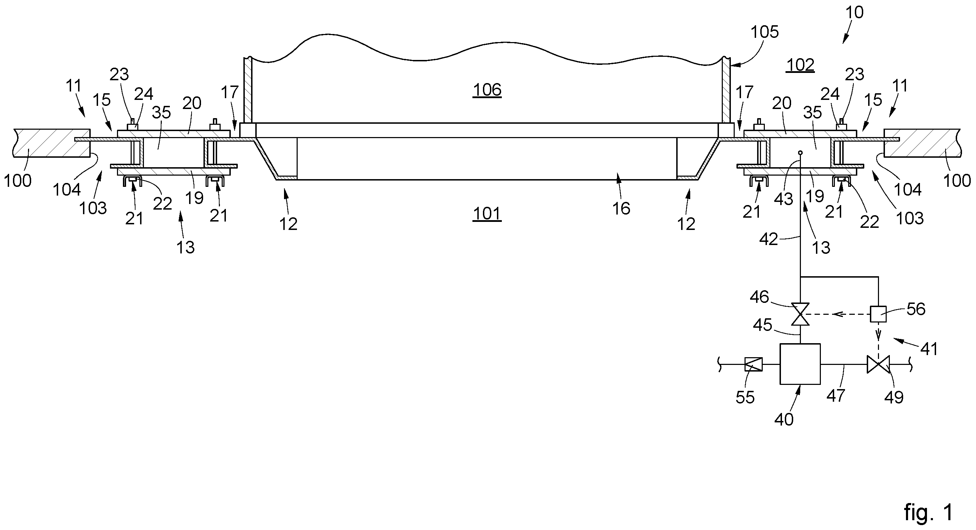

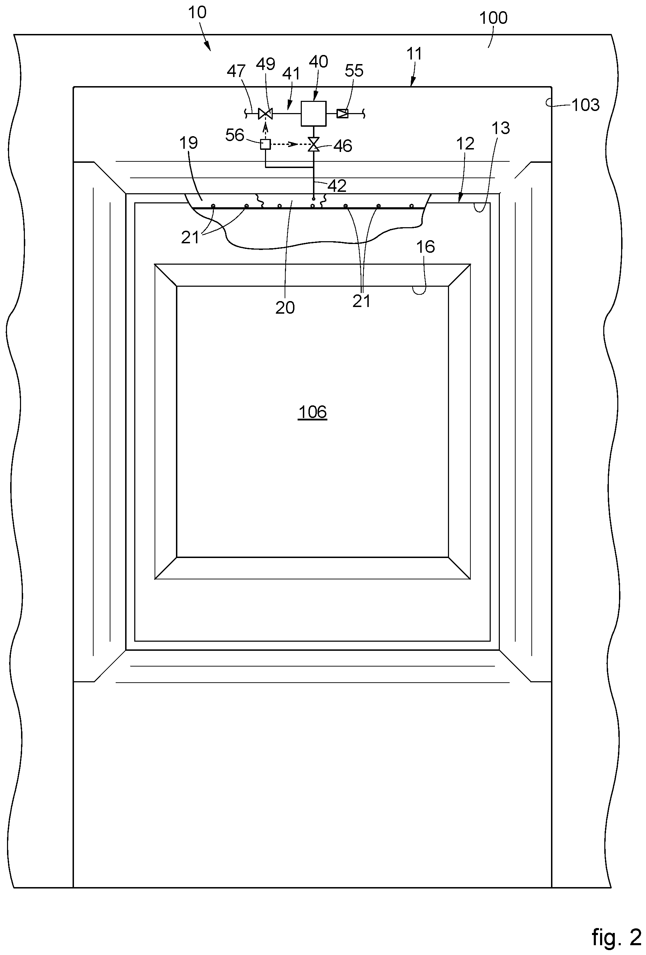

With reference to figs. 1 and 2, a hermetic sealing apparatus 10 according to the present invention is suitable and usable in a dividing wall 100 and has an interface function between a reception zone 101 for receiving contaminated instruments to be treated, and a sterile zone 102.

The instruments can be surgical or laboratory instruments used for operations or experiments.

In the reception zone 101, the instruments may have already been pre-treated, for example by means of washing and/or thermo-disinfection, but they are still partly contaminated. For example, the instruments may be contaminated with hazardous biological agents that have to be contained in closed and hermetically sealed environments.

The sterile zone 102 comprises a treatment chamber 106 in which the instruments are treated, in particular sterilized and/or sanitized.

The treatment chamber 106 can be part of a sterilization machine 105. For example, the sterilization machine 105 can be an autoclave comprising the treatment chamber 106 in which the contaminated instruments, possibly pre-treated, are sterilized using high temperature steam.

The dividing wall 100 is provided with a main aperture 103 delimited by main edges 104.

The sealing apparatus 10 comprises a support frame 11 perimetrically and hermetically attached to the main edges 104. The support frame 11 is provided with a first central aperture 13, delimited by first perimeter edges 15.

The sealing apparatus 10 also comprises a central rim 12 perimetrically and hermetically associated with the first perimeter edges 15 and provided with second perimeter edges 17 facing the latter. The central rim 12 comprises a second central aperture 16 which is hermetically connected to the treatment chamber 106 to allow the introduction of the contaminated instruments.

It should be noted that the treatment chamber 106 can be provided at entry with suitable hermetic closure means, not shown in the drawings, configured to selectively close the treatment chamber 106 itself with respect to the reception zone 101.

The first 15 and second 17 edges are reciprocally facing and spaced apart from each other, so that a hollow space 18 is formed between them.

Two sealing gaskets 19, 20 are attached between the first 15 and second 17 edges, also called bio-containment gaskets, which are disposed in series in the hollow space 18 and engaged pack-wise by corresponding attachment elements 21 cooperating with the corresponding first 15 and second 17 edges.

A hermetically closed isolation chamber 35 is created between the two gaskets 19, 20, which is able to prevent the transfer of contaminating agents from the reception zone 101 to the sterile zone 102.

Each attachment element 21 comprises a head, or proximal, end 22 and a terminal, or distal, end 23.

The terminal end 23 can cooperate with a clamping element 24 that stabilizes the attachment of the gaskets 19, 20.

The attachment element 21 can be a bolt and the clamping element 24 can be a nut. It is clear that other suitable attachment means can be used to attach the gaskets 19, 20 to the first 15 and second 17 edges.

Preferably, there is a first 19 and a second 20 gasket, wherein the second gasket 20 is disposed in, or faces toward, the sterile zone 102.

The gaskets 19, 20 have a rim-like shape and comprise respective external perimeter zones 19a, 20a attached to the first edges 15 and internal perimeter regions 19b, 20b attached to the second edges 17.

According to an embodiment shown in figs. 1, 2 and 3, the first 15 and second 17 edges can have respective profiles each comprising an undercut 25, 27 protruding toward the reception zone 101 and defining respective abutment portions 25a, 25b, 27a, 27b for the corresponding external 19a, 20a and internal 19b, 20b perimeter zones which are clamped from one side to the other by the attachment elements 21.

Each undercut 25, 27 is conformed so as to have a C-shaped profile, wherein the two respective abutment portions 25a, 25b and 27a, 27b are parallel and spaced apart from each other.

The abutment portions 25a, 25b of the undercut 25 of the first edges 15 are aligned with corresponding abutment portions 27a, 27b of the undercut 27 of the second edges 17 to form respective positioning planes P1, P2, parallel and spaced apart from each other, of the gaskets 19, 20. In this embodiment, the first gasket 19 lies on the first positioning plane P1 and the second gasket 20 lies on the second positioning plane P2. The two gaskets 19, 20 can have the same perimeter sizes.

Furthermore, the abutment portions 25a, 27a defining the first positioning plane P1 are positioned and facing toward the reception zone 101 and the abutment portions 25b, 27b defining the second positioning plane P2 are positioned and facing toward the sterile zone 102.

In this way, the head ends 22 of the attachment elements 21 are positioned in the reception zone 101 and the terminal ends 23 are positioned in the sterile zone 102.

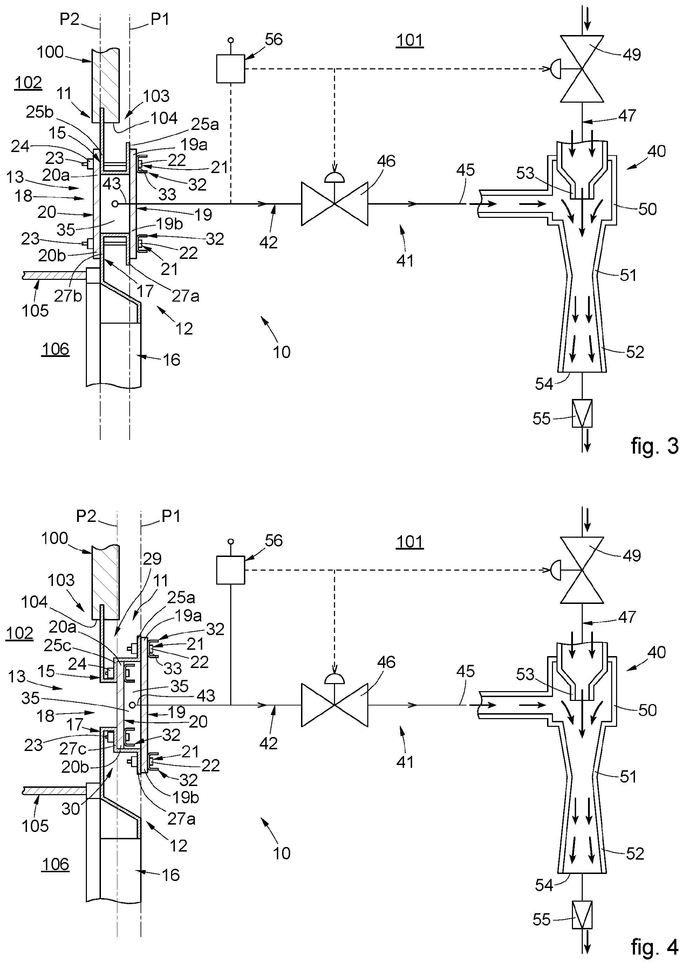

According to another embodiment shown in fig. 4, the first 15 and second 17 edges can have respective profiles each comprising at least two consecutive undercuts 25 and 27, protruding toward the reception zone 101 and defining respective abutment portions 25a, 25c, 27a, 27c for the corresponding external 19a, 20a and internal 19b, 20b perimeter zones which are clamped from one side to the other by the attachment elements 21, so that corresponding head 22 and terminal 23 ends of the latter remain in the reception zone 101.

This offers the advantage that even if there is not a perfect seal between the gaskets 19, 20 and the head 22 or terminal 23 ends of the clamping elements 21, any contaminating agents are not dispersed in the sterile zone 102, but remain in the reception zone 101. Another advantage is that open and non-blind nuts can also be used as clamping elements 24.

The two undercuts 25 and 27 are made so as to converge from the reception zone 101 toward the sterile zone 102. Therefore, the hollow space 18 has a greater width on the side of the reception zone 101 and a smaller width on the side of the sterile zone 102.

The undercuts 25 and 27 define, in the reception zone 101, on opposite sides with respect to the hollow space 18, respective collection compartments 29 and 30 that allow to contain any leakages of contaminating agents from the terminal ends 23 of the attachment elements 21.

Moreover, the two undercuts 25 and 27 are conformed so as to form a respective stepped profile, in which a rest part of each step defines a corresponding abutment portion 25a, 25c, 27a, 27c. The rest part is parallel to the dividing wall 100.

The abutment portions 25a, 25c of the undercuts 25 of the first edges 15 are aligned with corresponding abutment portions 27a, 27c of the undercuts 27 of the second edges 17 to form the respective positioning planes P1, P2, parallel and spaced apart from each other, of the gaskets 19, 20. In this embodiment, the first gasket 19 lies on the first positioning plane P1 and has larger perimeter sizes than the second gasket 20, which lies on the second positioning plane P2.

According to other embodiments, the first 15 and second 17 edges can have profiles each comprising three or more consecutive undercuts 25, 27, as defined above, to allow the disposition of as many sealing gaskets, which are clamped from one side to the other by the attachment elements 21 so that the corresponding head 22 and terminal 23 ends of the latter remain in the reception zone 101.

The sealing apparatus 10 comprises a plurality of tightening rods 32 which have a U-shaped cross section and a rest base 33. The tightening rods 32 are associated with the gaskets 19, 20 so that the respective base 33 is interposed between the head end 22 of each attachment element 21 and the corresponding external 19a, 20a and internal 19b, 20b perimeter zone of the gaskets 19, 20.

The sealing apparatus 10 comprises a device 40 for generating a vacuum through Venturi and a fluidic circulation circuit 41 connected to each other (figs. from 1 to 4). The vacuum generating device 40 and the circuit 41 can be disposed in the reception zone 101.

The circuit 41 comprises a first duct 42 hermetically associated with the chamber 35 and able to fluidically connect the latter to the vacuum generating device 40 in order to generate a negative pressure therein, able to draw any infiltrations of contaminating agents, expelling them into the reception zone 101.

The first duct 42 comprises a first end 43 hermetically associated with the chamber 35 and a second end 45 hermetically associated with the vacuum generating device 40. The first end 43 has an initial portion inserted inside the chamber 35.

A first valve 46 is disposed along the first duct 42, able to open and close for the selective fluidic connection between the chamber 35 and the vacuum generating device 40.

Moreover, the circuit 41 also comprises a second duct 47 connected to the vacuum generating device 40. The second duct 47 is disposed perpendicular with respect to the first duct 42.

A second valve 49 is disposed along the second duct 47, able to open and close for the selective passage of a driving fluid, preferably compressed air, toward the vacuum generating device 40. The introduction of compressed air is necessary for the correct Venturi effect.

The vacuum generating device 40 comprises a connection chamber 50, to which the first 42 and second 47 duct are hermetically connected, perpendicular to each other.

The vacuum generating device 40 (figs. 3 and 4) comprises a mixing part 51 connected to the connection chamber 50. The vacuum generating device 40 comprises a divergent part 52 from which a mixing fluid flows out into the reception zone 101. The mixing part 51 first converges in the direction of the flow of compressed air, then widens toward the divergent part 52. The mixing of the fluids coming from the first 42 and second 47 duct occurs in the connection chamber 50 and in the mixing part 51, in order to obtain the mixing fluid that flows out into the reception zone 101 through the divergent part 52.

Specifically, the second duct 47 comprises a narrowing nozzle 53 which is inserted inside the connection chamber 50 and which, thanks to the section narrowing, allows to increase the speed of the flow of compressed air toward the mixing zone 51, in order to promote the Venturi effect.

A silencing member 55 (figs. from 1 to 4), for example a silencing filter, can be advantageously disposed in correspondence with a terminal portion 54 of the divergent part 52, able to significantly reduce the noise generated by the outflowing fluid.

The sealing apparatus 10 comprises a pressure management device 56 fluidically associated with the first duct 42, between the chamber 35 and the first valve 46. The pressure management device 56 is operatively connected to the first 46 and second 49 valve.

The pressure management device 56 is configured to open and close the first 46 and second 49 valve for the selective activation of the vacuum generating device 40. In particular, the pressure management device 56 is configured to be activated as a function of an activation threshold value correlated to a pressure present in the chamber 35.

Advantageously, the pressure management device 56 is able to open and close the first 46 and second 49 valve simultaneously. This increases the speed and efficiency of the vacuum generator 40.

For example, the pressure management device 56 can comprise a control unit, not visible in the drawings, able to control the operation of the first 46 and second 49 valve.

According to preferred embodiments, the pressure management device 56 is a differential pressure switch able to be activated as a function of an activation threshold value given by a differential between a pressure present in the chamber 35 and a pressure present in the reception zone 101.

Alternatively, the pressure management device 56 can be another type of device, able to be activated as a function of an absolute activation threshold value correlated to the pressure present in the chamber 35.

Moreover, the pressure management device 56 is capable of generating a maintenance intervention alarm if the vacuum generating device 40, after a certain pre-established interval of time, is unable to restore a suitable pressure in the chamber 35. We must specify that the alarm can be a light and/or sound alarm signal. Alternatively, the alarm can be transmitted to a central control unit, not visible in the drawings.

We must specify that the suitable pressure has to be such as to result in the deactivation of the pressure management device 56 itself. Alternatively, it will be necessary to intervene in correspondence with the gaskets 19, 20 to check the seal of the gaskets 19, 20 and the isolation of the chamber 35. Advantageously, the sealing apparatus 10 allows to guarantee a biosafety level 4 (BSL-4).

The operation of the sealing apparatus 10 described heretofore, which corresponds to the method according to the present invention, comprises the following steps.

In a step, or condition, of correct hermetic seal, the pressure management device 56 is deactivated, the first 46 and second 49 valve are closed, and the vacuum generating device 40 is also deactivated.

In a vacuum generation step, the pressure management device 56 is activated as a function of an activation threshold value and determines the opening of the first 46 and second 49 valve, therefore the vacuum generating device 40 is also activated.

Specifically, the vacuum generating device 40 generates, through Venturi effect, a negative pressure in the chamber 35 in order to draw any infiltrations of contaminating agents and expel them into the reception zone 101.

The method also comprises a signaling step in which, if the vacuum generating device 40 fails to restore, after a certain pre-established interval of time, a suitable pressure inside the chamber 35, the pressure management device 56 generates a maintenance intervention alarm, to verify the seal in correspondence with the gaskets 19, 20.

It is clear that modifications and/or additions of parts may be made to the sealing apparatus 10 as described heretofore, without thereby departing from the field and scope of the present invention, as defined by the claims.

It is also clear that, although the present invention has been described with reference to some specific examples, a person of skill in the art will be able to achieve other equivalent forms of sealing apparatuses and corresponding operating methods, having the characteristics as set forth in the claims and hence all coming within the field of protection defined thereby.

In the following claims, the sole purpose of the references in brackets is to facilitate their reading and they must not be considered as restrictive factors with regard to the field of protection defined by the claims.

## Claims

1. Sealing apparatus (10) comprising both a support frame (11) provided with a first aperture (13) delimited by first perimeter edges (15) and able to be perimetrically and hermetically attached to a dividing wall (100) which separates a reception zone (101) for receiving contaminated instruments to be treated from a sterile zone (102), and also a central rim (12) provided with second edges (17) facing said first perimeter edges (15) with which it is perimetrically and hermetically associated, and with a second aperture (16) hermetically connectable to a treatment chamber (105) of said sterile zone (102), wherein between said first (15) and second (17) edges at least two sealing gaskets (19, 20) are attached, disposed in series and engaged by attachment elements (21) so as to define a hermetically closed isolation chamber (35) between them, said sealing apparatus (10) comprising a device (40) for generating a vacuum and a fluidic circulation circuit (41) having a first duct (42) hermetically associated with said chamber (35) and able to connect it fluidically to said vacuum generating device (40) in order to generate a negative pressure therein able to draw any infiltrations of contaminating agents, expelling them into said reception zone (101), **characterized in that** said vacuum generating device (40) generates said negative pressure through the Venturi effect and said sealing apparatus (10) comprises: a first valve (46) disposed along said first duct (42) and able to open and close for the selective fluidic connection between said chamber (35) and said vacuum generating device (40); a second valve (49) disposed along a second duct (47) connected to said vacuum generating device (40) and able to open and close for the selective passage of a driving fluid toward the latter; and a pressure management device (56) fluidically associated with said first duct (42) between said chamber (35) and said first valve (46) and operationally connected to said first (46) and second (49) valve, wherein said pressure management device (56) is configured to open and close said first (46) and second (49) valve simultaneously, for the selective activation of said vacuum generating device (40).

2. Sealing apparatus (10) as in claim 1, **characterized in that** said pressure management device (56) is configured to activate as a function of an activation threshold value correlated to a pressure present in said chamber (35).

3. Sealing apparatus (10) as in any claim hereinbefore, **characterized in that** said pressure management device (56) is a differential pressure switch able to activate as a function of an activation threshold value given by a differential between a pressure in said chamber (35) and a pressure in said reception zone (101).

4. Sealing apparatus (10) as in any claim hereinbefore, **characterized in that** said pressure management device (56) is capable of generating a maintenance intervention alarm if said vacuum generating device (40) is not capable of restoring, after a certain interval of time, a suitable pressure in said chamber (35).

5. Sealing apparatus (10) as in any claim hereinbefore, **characterized in that** said second duct (47) comprises a narrowing nozzle (53) which is inserted inside a connection chamber (50) to which the first (42) and second (47) duct are hermetically connected and which, thanks to the section narrowing, allows to increase the speed of the flow of the driving fluid toward a mixing zone (51) in order to promote the Venturi effect.

6. Sealing apparatus (10) as in any claim hereinbefore, **characterized in that** said vacuum generating device (40) comprises a divergent part (52) from which a mixing fluid flows out into said reception zone (101).

7. Sealing apparatus (10) as in claim 6, **characterized in that** a silencing member (55) is positioned in correspondence with a terminal portion (54) of said divergent part (52).

8. Sealing apparatus (10) as in any claim hereinbefore, **characterized in that** said first (15) and second (17) edges have profiles comprising an undercut (25, 27) protruding toward the reception zone (101) and having respective abutment portions (25a, 25b, 27a, 27b) for corresponding external (19a, 20a) and internal (19b, 20b) perimeter zones of said gaskets (19, 20) which are clamped from one side to the other by said attachment elements (21), so that one of said two gaskets (19, 20) is disposed and facing toward said sterile zone (102) and that corresponding head (22) and terminal (23) ends of said attachment elements (21) are present in said reception zone (101) and in said sterile zone (102), respectively.

9. Sealing apparatus (10) as in any claim from 1 to 7, **characterized in that** said first (15) and second (17) edges have profiles each comprising at least two consecutive undercuts (25, 27), protruding toward said reception zone (101) and defining respective abutment portions (25a, 25c, 27a, 27c) for corresponding external (19a, 20a) and internal (19b, 20b) perimeter zones of said gaskets (19, 20) which are clamped from one side to the other by said attachment elements (21) so that corresponding head (22) and terminal (23) ends of the latter remain in said reception zone (101).

10. Sealing apparatus (10) as in claim 9, **characterized in that** said at least two undercuts (25, 27) of said first (15) and second (17) edges are spaced apart from each other, defining a hollow space (18) in which said gaskets (19, 20) are disposed, and are created in such a way as to define stepped profiles converging from said reception zone (101) toward said sterile zone (102) so that said hollow space (18) has a greater width toward the reception zone (101) and a smaller width toward the sterile zone (102).

11. Sealing apparatus (10) as in claim 10, **characterized in that** in the reception zone (101), on opposite sides with respect to the hollow space (18), said undercuts (25, 27) define respective collection compartments (29, 30) which allow to contain any leakages of contaminating agents from the terminal ends (23) of the attachment elements (21).

12. Method of operation of a hermetic sealing apparatus (10) as in any claim hereinbefore, comprising a step of generating a vacuum in said chamber (35), in which said vacuum generating device (40) generates a negative pressure in said chamber (35) through Venturi in order to draw any infiltrations of contaminating agents present therein and expel them into said reception zone (101), wherein in said vacuum generation step a pressure management device (56) is activated and opens a first (46) and a second (49) valve, thus activating said vacuum generating device (40), wherein said first valve (46) is disposed along said first duct (42) and opens and closes for the selective fluidic connection between said chamber (35) and said vacuum generating device (40), and wherein said second valve (49) is disposed along a second duct (47) connected to said vacuum generating device (40) and opens and closes for the selective passage of a driving fluid toward the latter.
